# EUROPÄISCHE PATENTANMELDUNG

(11) **EP 0 649 015 A1**
(43) Veröffentlichungstag der Anmeldung: **19.04.1995**
(21) Anmeldenummer: 94810596.0
(22) Anmeldetag: 13.10.1994
(51) Int. Cl.: G01N 21/27, A23C 9/15, G01N 33/06

(54) **Verfahren zur Durchführung eines automatischen, periodischen Nullpunktabgleichs**

(30) Priorität: 14.10.1993 CH 3108/93
(71) Anmelder: APV (Schweiz) AG, CH-3076 Worb (CH)
(72) Erfinder: Meier, Roger, CH-3638 Blumenstein / BE (CH)
(74) Vertreter: Roshardt, Werner Alfred, Dipl.-Phys.

(57) **Zusammenfassung**

In einer Anlage zum Standardisieren von Milch werden Messdifferenzen (d₁ ... d₁₂) im Sinne eines periodischen, automatischen Nullpunktabgleichs kompensiert. Die zu kompensierende Differenz (d₁ ... d₁₂) wird mit einem vordefinierten Toleranzbereich (Bₒ, Bᵤ) verglichen. Liegt sie innerhalb dieses Bereichs, wird sie bis zur Durchführung des nächsten Nullpunktabgleichs als Basis für die rechnerische Messwertkompensation herangezogen. Liegt sie (d₇, d₁₀) ausserhalb des Toleranzbereichs (Bₒ, Bᵤ), so wird stattdessen ein auf der Basis vergangener, innerhalb des vordefinierten Toleranzbereichs (Bₒ, Bᵤ) gelegener Differenzen (d₁ ... d₁₂) ermittelter Sicherheitswert (S₇, S₁₀) verwendet.

## Beschreibung

### Technisches Gebiet

Die Erfindung betrifft ein Verfahren zur Durchführung eines automatischen, periodischen Nullpunktabgleichs von Messwerten in einem mehrere Medien verarbeitenden Prozess, wobei mindestens eine Messzelle zur Ermittlung des Nullpunktabgleichs in periodischen Messintervallen statt mit dem jeweils zu überwachenden Medium mit einem Referenzmedium beschickt wird und der für das Referenzmedium gemessene Wert mit mindestens einer gespeicherten Grösse verglichen und daraus der Nullpunktabgleich ermittelt wird. Ferner bezieht sich die Erfindung auf eine Anwendung des Verfahrens und eine Vorrichtung zur Durchführung des Verfahrens.

### Stand der Technik

Ein Verfahren der eingangs genannten Art ist z. B. aus der DE-OS 41 39 380 bekannt. Die entsprechende Technik geht zurück auf die in der CH-PS 593 618 beschriebene Standardisieranlage.

Es werden dabei die Dichten von Rahm, Magermilch und standardisierter Milch gemessen und daraus der Fettgehalt der genannten Medien ermittelt. Das Funktionsprinzip basiert auf den ermittelten Dichtedifferenzen zwischen Magermilch, standardisierter Milch und Rahm. Die Dichten der einzelnen Medien werden im Bypass zum jeweiligen Hauptstrom mit hochgenauen Prozessdichtemesszellen erfasst. Die Dichtemesszellen arbeiten nach dem Prinzip des schwingenden U-Rohres (Coriolisprinzip) und erlauben eine kontinuierliche Prozessdichtemessung mit höchster Genauigkeit.

Da die erwähnte Anlage mit der Referenzdichte zu Magermilch arbeitet, ist sie im Prozess immer mit einem Separator gekoppelt, welcher Vollmilch in Rahm und Magermilch separiert. Die optimale Separationstemperatur für Milch liegt bei ca. 55-60 °C.

Bei diesen Prozesstemperaturen erfolgt bei bestimmten hitzelabilen Molkenproteinfraktionen (vor allem Immunoglobulinen, Blutserumalbumin, Rinderserumalbumin) bereits eine Hitzedenaturierung. Diese Hitzedenaturierung gewisser Molkenproteine äussert sich in der Milch in Form sehr kleiner, mit dem Auge nicht sichtbarer Flöckchen, die sich zusammen mit Calciumphosphat [Ca₃(PO₄)₂] vor allem im Erhitzerabteil des Pasteurs, aber auch im gesamten Rohrleitungssystem ablagern.

Aufgrund des Anlageprinzips findet man diese Proteinablagerungen in sehr geringen Mengen auch in den Prozessdichtemesszellen (vor allem in der Magermilchmesszelle), was im Verlaufe mehrerer Prozessstunden neben anderen Faktoren zu Messungenauigkeiten führen kann (langsames Abdriften des Ist-Wertes).

Von der aus der CH-PS 593 618 bekannten Anlage unterscheidet sich die in der DE 41 39 380 A1 beschriebene dadurch, dass in der Anlage nicht drei (nämlich für Magermilch, standardisierte Milch und Rahm je eine), sondern insgesamt nur eine Messzelle vorgesehen ist. In dieser einzigen Messzelle wird primär die Dichte (und damit der Fettgehalt) der standardisierten Milch gemessen. In bestimmten Abständen wird die Messzelle mit Magermilch beschickt, um gegebenenfalls eine Aenderung des Referenzwertes (Magermilchdichte) zu registrieren.

Im Prozess können Störungen auftreten, die zu tatsächlichen Abweichungen der Dichten führen (z. B. Restluft). Derartige Störungen führen bei der aus der DE-OS 41 39 380 bekannten Anlage zu einem Alarm, welcher aber eigentlich nicht auf einer Fehlfunktion der Messzelle beruht. Entsprechend wird das dort vorgeschlagene Auswaschen der Messzellen unnötigerweise durchgeführt.

### Darstellung der Erfindung

Aufgabe der Erfindung ist es nun, ein Verfahren der eingangs genannten Art anzugeben, das auch bei Störungen, die nicht durch ablagerungsbedingte Messfehler hervorgerufen werden, in geordneter Weise weiterarbeitet. Insbesondere sollen unnötige und undifferenzierte Alarme vermieden werden.

Gemäss der Erfindung besteht die Lösung darin, dass - falls die Differenz zwischen gemessenem und zwischengespeichertem Wert ausserhalb eines vordefinierten Toleranzbereiches liegt - der Nullpunktabgleich auf der Basis eines statistischen Sicherheitswerts vergangener, innerhalb des vordefinierten Toleranzbereichs liegender Differenzen ermittelt wird.

Der Toleranzbereich definiert die Bandbreite ablagerungsbedingter Messfehler (reguläre Drift). Liegt eine Differenz ausserhalb dieses Bereichs, wird sie für den zukünftigen Nullpunktabgleich nicht herangezogen. Vielmehr wird z. B. ein statistischer Mittelwert vergangener zulässiger Differenzen zur Ermittlung des Nullpunktabgleichs herangezogen.

Die gespeicherte Grösse kann ein früher gemessener Wert derselben Messzelle (wie z. B. in der DE-OS 41 39 380) oder ein im selben Messzyklus gemessener Wert einer anderen Zelle (wie in der CH-PS 593 618) sein.

Vorzugsweise wird der genannte Toleranzbereich bei Inbetriebnahme der Anlage vorgegeben. Er kann dabei ein für allemal festgelegt sein. Denkbar ist auch, dass er in grösseren Zeitabständen unter Zuhilfenahme statistischer Methoden aus den bisher gemessenen Differenzwerten neu berechnet wird.

Falls die Differenz innerhalb des Toleranzbereichs liegt, wird der Nullpunktabgleich vorzugsweise nur auf der Basis der zuletzt ermittelten Differenz bestimmt. D. h., es werden keine anderen resp. früheren Werte berücksichtigt.

Gemäss einer besonders bevorzugten Ausführungsform ist für jedes Medium eine separate Messzelle zwecks kontinuierlicher Ueberwachung vorgesehen. Innerhalb des Messintervalls werden alle Messzellen einmal mit dem Referenzmedium beschickt. Die entsprechenden Werte werden zwischengespeichert, die Differenzen zwischen den Werten verschiedener Messzellen gebildet und mit dem entsprechenden Toleranzbereich verglichen. Auf diese Weise kann jedes Medium kontinuierlich überwacht werden. Während des Nullpunktabgleichs werden die den Prozess regelnden Stellgrössen kurzzeitig festgehalten (blockiert).

Als Referenzmedium kann eines der im Prozess verarbeiteten Medien verwendet werden. Wird das Nullpunktabgleichsverfahren in einem Prozess zum Standardisieren von Milch angewendet, dann wird als Referenzmedium vorzugsweise Magermilch benützt.

Vorzugsweise wird das Verfahren bei einer Vorrichtung gemäss CH-PS 593 618 eingesetzt. Eine solche Vorrichtung umfasst einen Separator zum Auftrennen von Vollmilch in Rahm und Magermilch, eine Verbindungsleitung zum Zudosieren von Rahm in die Magermilch, einen Mischer zum Vermischen von Magermilch und zudosiertem Rahm zu standardisierter Milch und drei Messzellen zum Messen der Dichte des Rahms, der Magermilch und der standardisierten Milch. Alle drei Messzellen sind umschaltbar mit einer die Magermilch führenden Leitung verbunden zur Durchführung des erfindungsgemässen Nullpunktabgleichs mit Ueberwachung.

Das erfindungsgemässe Verfahren wird vorzugsweise mit einer digitalen Steuerung realisiert. Die zur Durchführung des erfindungsgemässen Verfahrens geeigneten Programme sind in einem Festspeicher (z. B. EEPROM) enthalten. Die zu speichernden Messwerte werden in an sich bekannter Weise in einem RAM abgespeichert. Die Art und Weise der Umsetzung des Verfahrens in ein geeignetes Programm ist dem Fachmann geläufig.

### Kurze Beschreibung der Zeichnungen

Nachfolgend soll die Erfindung anhand eines Ausführungsbeispiels und im Zusammenhang mit den Zeichnungen erläutert werden. Sie zeigen:

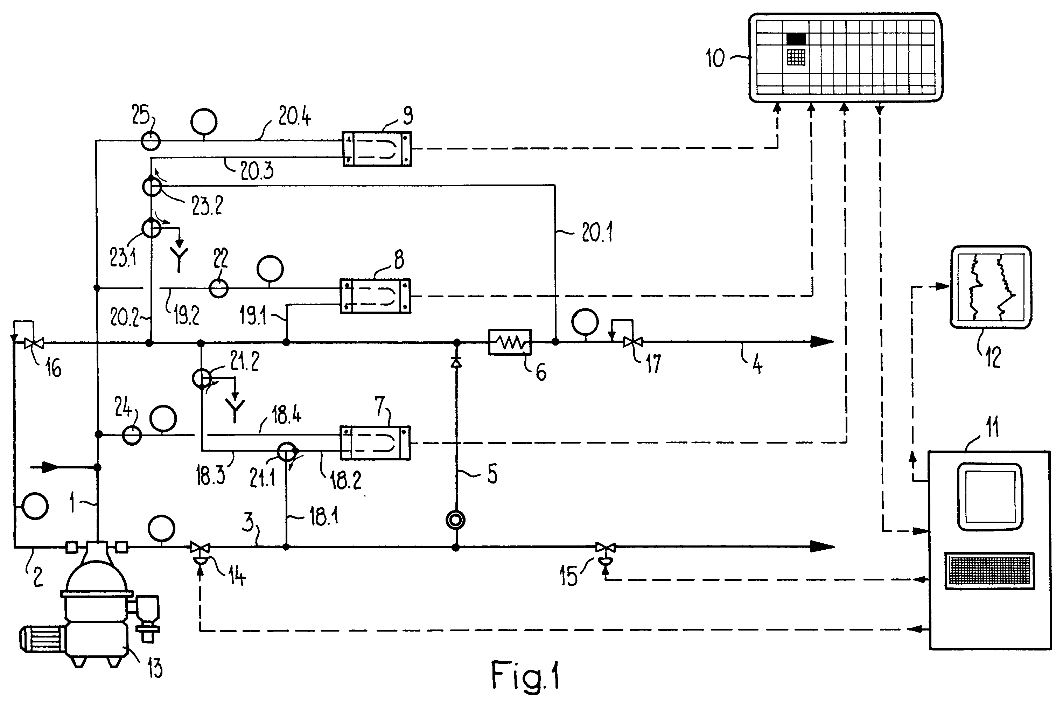

- Fig. 1: ein Flussdiagramm einer erfindungsgemässen Anlage;
- Fig. 2: eine Prinzipdarstellung der erfindungsgemässen Plausibilitätsprüfung.

### Wege zur Ausführung der Erfindung

Das Verfahren zur Aufbereitung der Vollmilch in standardisierte Milch und standardisierten Rahm ist vom Grundprinzip her aus der CH-PS 593 618 bekannt. An dieser Stelle soll deshalb nur das wesentliche des Verfahrens und der entsprechenden Anlage erläutert werden.

Ueber eine Vollmilchleitung 1 wird die Vollmilch unter äusserst konstantem Druck einem Separator 13 (Zentrifuge) zugeführt. Dieser trennt die Vollmilch in Rahm (Rahmleitung 3) und Magermilch (Magermilchleitung 2). Rahmleitung 3 und Magermilchleitung 2 sind über eine Rahmzudosierleitung 5 verbunden, um die Magermilch mit einem genau bestimmten Fettanteil anzureichern. Ein Mischer 6 homogenisiert das Magermilch/Rahm-Gemisch zu standardisierter Milch (Standardmilchleitung 4). Die Separationsgrenze des Separators 13 wird durch Steuerung der Ausgangsdruckverhältnisse mit einem Regelventil 14 in der Rahmleitung 3 und einem Gegendruckventil 16 in der Magermilchleitung 2 eingestellt. Mit Hilfe von Messzellen 7 und 8 wird der Fettgehalt des Rahms und der Magermilch vor der Verzweigung zur Rahmzudosierleitung 5 ermittelt. Die zudosierte Rahmmenge wird durch ein nachgeschaltetes Regelventil 15 gesteuert. Hinter dem statischen Mischer 6 befindet sich ein Gegendruckventil 17 zur Aufrechterhaltung eines konstanten Drucks. Eine Messzelle 9 ist zwischen Mischer 6 und Gegendruckventil 17 an die Standardmilchleitung 4 angeschlossen.

Ein Prozessrechner 10 (Slave) erfasst die Messwerte der Messzellen 7, 8, 9 und errechnet daraus in an sich bekannter Weise kontinuierlich den entsprechenden Fettgehalt. Er (bzw. ein überlagerter Rechner) steuert und kontrolliert auch den erfindungsgemässen Nullpunktabgleich. Der Prozessrechner 10 kommuniziert mit einem Steuerschrank 11 (Leitrechner), welcher die Regelventile 14 und 15 kontrolliert. Ein Prozessdatenschreiber 12 zeichnet zur Kontrolle z. B. die registrierten Fettgehalte auf.

Gemäss einer besonders bevorzugten Ausführungsform der Erfindung sind die Messzellen 7 und 9 über Bypassleitungen 18.1, 18.2 resp. 20.1, 20.3 nicht nur eingangsseitig an die Rahmleitung 3 resp. Standardmilchleitung 4, sondern zusätzlich auch über Bypassleitungen 18.3 resp. 20.2 an die Magermilchleitung 2 angeschlossen. Ausgangsseitig sind beide Messzellen 7, 9 an die unter sehr konstantem Druck stehende Vollmilchleitung 1 angeschlossen (Bypassleitungen 18.4 resp. 20.4). Die Messzelle 8 ist über Bypassleitungen 19.1, 19.2 nur an die Magermilch- und Vollmilchleitungen 2 resp. 1 angeschlossen.

Das Umschalten der Messzellen 7 und 9 von Rahm bzw. standardisierter Milch auf Magermilch erfolgt durch automatisch ansteuerbare Dreiweghähne 21.1, 21.2 resp. 23.1, 23.2 und Ventile 24 resp. 25. In der Bypassleitung 19.2 ist in sinngemässer Weise ein Ventil 22 vorgesehen.

In der Regel messen die Messzellen 7, 8, 9 kontinuierlich die Dichte von Rahm, Magermilch resp. standardisierter Milch. In vorgegebenen Messintervallen werden die Messzellen 7 und 9 jedoch mit Magermilch beschickt zur Durchführung eines Nullpunktabgleichs.

Bei der Nullpunktauslösung wird zuerst die Stellgrösse des Milch- bzw. Rahmreglers (Regelventile 14, 15) auf dem aktuellen Wert blockiert. Um bei kleinen Regeldifferenzen ein unnötiges Aufintegrieren zu vermeiden, wird der Integrationsanteil im Regelalgorithmus während der Nullpunktermittlung ausgeschaltet. Zeitverzögert werden die Messzellen 7 und 9 mit Magermilch beschickt. Die Differenzen zwischen den von den Messzellen 7 bzw. 8 und 8 bzw. 9 ermittelten Dichten der Magermilch werden zwischengespeichert. Anschliessend werden die Messzellen 7, 9 wieder mit ihren üblichen Medien (Rahm bzw. standardisierte Milch) beschickt und die Reglerstellgrössen wieder freigegeben.

In einer zweiten Stufe geht es nun darum, die Differenzen auf ihre Plausibilität zu überprüfen und den rechnerischen Abgleich der drei Messzellen 7, 8, 9 zu ermitteln. Zu diesem Zweck wird jede Differenz mit einem vordefinierten, symmetrischen oder asymmetrischen Toleranzband verglichen. Liegt sie innerhalb desselben, so wird sie bis zur Durchführung des nächsten Nullpunktabgleichs als Basis für den rechnerischen Abgleich der Messwerte der entsprechenden Messzelle benützt. Ist sie dagegen ausserhalb der Toleranzbandes, dann wird sie verworfen. Stattdessen wird bis zur nächsten Ermittlung des Nullpunktabgleichs ein statistischer Mittelwert vergangener, innerhalb des Toleranzbandes gelegener Messwerte als Basis für den rechnerischen Abgleich des Nullpunkts verwendet.

Die innerhalb des Toleranzbandes liegenden Differenzen werden in einem dynamischen RAM abgespeichert und in der folgenden Messperiode zyklisch alle 100 ms in die Fettgehaltsberechnung einbezogen.

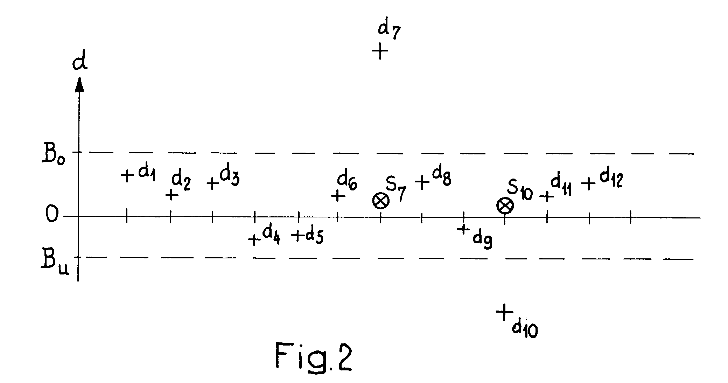

Das Gesagte soll anhand der Fig. 2 beispielhaft erläutert werden. d₁ ... d₁₂ bezeichnet eine Reihe ermittelter Differenzen beispielsweise zwischen den Messzellen 7 und 8. Idealerweise müssten diese Differenzen alle verschwinden (handelt es sich doch um die Dichte ein und desselben Mediums). In der Praxis treten nun aber beispielsweise die in Fig. 2 gezeigten Differenzen d₁ ... d₁₂ auf.

Der grösste Teil dieser Differenzen (nämlich alle bis auf d₇ und d₁₀) liegen innerhalb des vorgegebenen Toleranzbereiches, welcher nach oben durch Bₒ und nach unten durch Bᵤ abgegrenzt ist. Gemäss der Erfindung werden Differenzen, die innerhalb des Toleranzbereiches (Bₒ, Bᵤ) liegen, herangezogen, um bis zur nächsten Durchführung eines Nullpunktabgleichs die Unterschiede der in den beiden entsprechenden Messzellen kontinuierlich (100 ms) ermittelten Werte abzugleichen. Tritt eine Differenz auf (z. B. d₇), die ausserhalb des Toleranzbereichs (Bₒ, Bᵤ) liegt, dann wird der Nullpunktabgleich (bis zur nächsten Ermittlung der Differenz d₈) nicht auf der Basis der zu grossen Differenz d₇, sondern auf der Basis eines statistisch ermittelten Sicherheitswerts S₇ durchgeführt. Dieser Sicherheitswert S₇ ergibt sich beispielsweise aus einem gleitenden Mittelwert vergangener, innerhalb des Toleranzbereichs (Bₒ, Bᵤ) liegender Differenzen d₁ ... d₆ . Die ausserordentliche Differenz d₇ wird gelöscht. Sie kann also einen später ermittelten Sicherheitswert S₁₀ nicht beeinflussen, auch wenn sie innerhalb des für die Bildung des gleitenden Mittelwerts relevanten Zeitintervalls liegt.

Der Toleranzbereich kann symmetrisch oder asymmetrisch sein. Ferner wird für jedes Messzellenpaar ein separater Toleranzbereich anlagespezifisch definiert bzw. festgelegt.

Der erfindungsgemässe automatische, periodische Nullpunktabgleich eignet sich auch für ein Messsystem, wie es in der DE-OS 41 39 380 A1 beschrieben. Dort werden nicht Differenzen zwischen verschiedenen Messzellen, sondern Differenzen zu vorangegangenen Messwerten ermittelt. Liegen bei einer solchen Messanordnung die Differenzen aufeinanderfolgender Messwerte ausserhalb des erfindungsgemässen Toleranzbandes, dann wird im Rahmen der vorliegenden Erfindung (wie oben beschrieben) ein statistischer Sicherheitswert zur Berechnung des Fettgehalts herangezogen.

Die Erfindung beschränkt sich nicht auf Verfahren zur Herstellung von standardisierter Milch. Vielmehr kann sie auch in anderen Anlagen mit einer oder mehreren Messzellen zur Durchführung eines Nullpunktabgleichs herangezogen werden.

Durch die Erfindung wird eine beträchtliche Verbesserung der aus dem Stand der Technik bekannten Anlagen erzielt.

## Patentansprüche

1. Verfahren zur Durchführung eines automatischen, periodischen Nullpunktabgleichs von Messwerten in einem mehrere Medien verarbeitenden Prozess, wobei mindestens eine Messzelle zur Ermittlung des Nullpunktabgleichs in periodischen Messintervallen statt mit dem jeweils zu überwachenden Medium mit einem Referenzmedium beschickt wird und der für das Referenzmedium gemessene Wert mit mindestens einer gespeicherten Grösse verglichen und daraus der Nullpunktabgleich ermittelt wird, dadurch gekennzeichnet, dass, falls die Differenz (d₇) zwischen gemessenem Wert und gespeicherter Grösse ausserhalb eines vordefinierten Toleranzbereichs (Bₒ, Bᵤ) liegt, der Nullpunktabgleich auf der Basis eines statistischen Sicherheitswerts (S₇) aus vergangenen, innerhalb des vordefinierten Toleranzbereichs (Bₒ, Bᵤ) liegenden Differenzen (d₁ ... d₆) ermittelt wird.

2. Verfahren nach Anspruch 1, dadurch gekennzeichnet, dass, falls die Differenz (d₆) innerhalb des Toleranzbereichs (Bₒ, Bᵤ) liegt, der Nullpunktabgleich nur auf der Basis dieser Differenz (d₆) ermittelt wird.

3. Verfahren nach Anspruch 1 oder 2, dadurch gekennzeichnet, dass für jedes Medium eine separate Messzelle (7, 8, 9) verwendet wird, innerhalb des Messintervalls alle Messzellen (7, 8, 9) einmal mit dem Referenzmedium beschickt werden, die entsprechenden Werte zwischengespeichert werden, Differenzen zwischen den Werten verschiedener Messzellen (7 und 8 resp. 9 und 8) gebildet und mit dem entsprechenden Toleranzbereich (Bₒ, Bᵤ) verglichen werden zur Ermittlung des Nullpunktabgleichs.

4. Verfahren nach einem der Ansprüche 1 bis 3, dadurch gekennzeichnet, dass als Referenzmedium eines der verarbeiteten Medien verwendet wird.

5. Anwendung des Verfahrens nach einem der Ansprüche 1 bis 4 in einem Prozess zum Standardisieren von Milch, wobei als Referenzmedium Magermilch eingesetzt wird.

6. Vorrichtung zur Durchführung des Verfahrens nach einem der Ansprüche 1 bis 4 mit mindestens einer Messzelle (7, 8, 9) und einem Prozessrechner (10) mit einem Programmspeicher enthaltend ein Programm zur Durchführung des Verfahrens und einem Datenspeicher zum Zwischenspeichern von Werten.

7. Vorrichtung zur Durchführung der Anwendung nach Anspruch 5 mit
a) einem Separator (13) zum Auftrennen von Vollmilch in Rahm und Magermilch,
b) einer Verbindungsleitung (5) zum Zudosieren von Rahm in die Magermilch,
c) einem Mischer (6) zum Vermischen von Magermilch und zudosiertem Rahm zu standardisierter Milch und
d) drei Messzellen (7, 8, 9) zum Messen der Dichte des Rahms, der Magermilch und der standardisierten Milch,
dadurch gekennzeichnet, dass
e) die in der Regel nicht mit Magermilch beschickten Messzellen (7, 9) umschaltbar mit einer die Magermilch führenden Leitung (2) verbunden sind zur automatischen Durchführung des Nullpunktabgleichs.
